# EUROPEAN PATENT SPECIFICATION

(11) **EP 2 714 149 B1**
(45) Date of publication and mention of the grant of the patent: **30.09.2015**
(21) Application number: 12726090.9
(22) Date of filing: 01.06.2012
(51) Int. Cl.: A61M 5/315, A61M 5/24, A61M 5/31, A61M 5/32

(54) **MEDICAL DEVICE HAVING USER FRIENDLY CONTROL INPUT**
MEDIZINISCHE VORRICHTUNG MIT BENUTZERFREUNDLICHER STEUEREINGABE
DISPOSITIF MÉDICAL AYANT UNE ENTRÉE DE COMMANDE CONVIVIALE

(30) Priority: 01.06.2011 EP 11168554; 09.06.2011 US 201161495115 P
(43) Date of publication of application: 09.04.2014
(73) Proprietor: Novo Nordisk A/S, 2880 Bagsværd (DK)
(72) Inventor: KJELDSEN, Bastian, Gaardsvig, DK-2880 Bagsværd (DK); NIELSEN, Karsten, Baker, DK-2880 Bagsværd (DK); MOURIDSEN, Brian, DK-2880 Bagsværd (DK)
(86) International application number: PCT/EP2012/060451
(87) International publication number: WO 2012/164097

(56) References cited:
- WO-A1-01/87386
- WO-A1-2010/084113

## Description

The present invention generally relates to medical devices adapted for managing medical therapy as well as components for use in such devices. In specific embodiments the invention relates to medical delivery devices and components therefore adapted to provide ease of use in a cost-effective way.

### BACKGROUND OF THE INVENTION

In the disclosure of the present invention reference is mostly made to the treatment of diabetes by delivery of insulin, however, this is only a preferred use of the present invention.

Conventional delivery devices for delivery of liquid drugs by means of subcutaneous injection typically have been provided as devices such as pen-shaped devices having a cylindrical form-factor. The cylindrical form-factor approach has mainly been chosen due to the particular use of cylindrical drug-filled cartridges. While the cylindrical form-factor generally enables design of slim devices, this form-factor typically has short-comings as regards device length and available surface area for presenting information to the user, e.g. by means of a display device.

To overcome these problems, delivery devices having other form-factors than the pen-shaped form-factor have been proposed. Examples of injection devices having both the cylindrical form-factor as well as devices having a non cylindrical form factor are disclosed in WO 98/10814.

Typically, the operation of the device is provided by means of various push-buttons and/or rotary selectors, e.g. a rotational wheel or drum for selecting among a plurality of different dose sizes. Some delivery devices, for example motorised dosers or infusion pumps, typically employ push-buttons instead of a rotary selector in order to dial up or dial down a dosage amount of medicament or a flow rate or in order to adjust other user selectable parameters. However, rotary selectors generally provide for a more intuitive operation and rotary selectors may be provided as well also in a motorised delivery device. Generally, from a users viewpoint there is a wish that the delivery device is as slim as possible in order to provide for a compact device which may be easily carried about, i.e. in a shirt-pocket or the like. In the device shown in fig. 3 of WO 98/10814, for rotary selectors which rotate about an axis parallel to a flat surface of the housing of the device, the reduced dimension orthogonal to the flat surface may lead to drawbacks as regards operability of the rotary selector. The diameter of the rotary selector may simply become too small to provide for an ergonomic grip. This may lead to difficulties in setting the correct dose. Other flat devices, such as the device shown in fig. 3 of WO 01/87386, propose to include a dosage selector which rotates about an axis orthogonal to the flat surfaces of the housing. A similar configuration in a device utilizing a flat flexible medicament reservoir is shown in WO 2006/003130. However, such design of the rotary selector may lead to compromises in the surface area on a flat area of the device housing which otherwise could be made available for presenting information. In addition, such design may present other drawbacks as regards the space needed for coupling the rotary selector to the drive mechanism and/or to encoders in the device.

Having regard to the above, it is a first object of the present invention to provide a medical delivery device having an operable control member which offers increased operability regardless of the particular shape and form-factor of the housing.

Generally, in order to provide medical devices combining user selectable parameters by means of one or more operable control members with a compact and pocket-friendly form-factor, it is a second object of the present invention to provide components for a medical device which allow for a compact design according to a non-cylindrical form factor without being associated with the deficiencies in operability often found in prior art medical devices.

### DISCLOSURE OF THE INVENTION

In the disclosure of the present invention, embodiments and aspects will be described which will address one or more of the above objects or which will address objects apparent from the below disclosure as well as from the description of exemplary embodiments.

In the above as well as in the following, reference will be made to drug injection devices, however, the present invention may be used in any device within the medical field in which similar situations of use give rise to similar problems.

**It should be noted that the appended set of claims corresponds to the below second aspect of the invention.**

Thus, in a **first aspect** of the invention a medical device is provided which comprises a housing and an operable control member arranged relative to the housing, wherein the operable control member is movable relative to an exterior surface section of the housing for altering a parameter relating to the operation of the medical device, wherein the operable control member comprises a belt-like structure arranged to move along the exterior surface section of the housing by means of a sliding movement in a direction along the extension of the belt-like structure. The section of the housing where the operable control member is located may include a non-circular outer surface and the belt-like structure may be configured to slide along the non-circular outer surface whereby. respective portions of the operable control member change radius of curvature as they move along the non-circular outer surface of the housing.

In a **second aspect** of the invention a medical delivery device is provided which comprises a) a housing adapted to accommodate a drug in a drug reservoir, the housing having an exterior surface defining at least one non-circular section, b) a drive mechanism for expelling doses of the drug from the drug reservoir, and c) an operable control member arranged at the non-circular section of the housing, the operable control member being movable relative to the non-circular section of the housing for altering a parameter relating to the operation of the medical delivery device. The operable control member comprises a belt-like structure arranged to move along the non-circular section of the housing so that respective portions of the operable control member change radius of curvature as they move along the non-circular section of the housing.

In a **third aspect** of the invention a needle magazine is provided, the needle magazine being, adapted for storing a plurality of injection needles for use with an associated medical delivery device. The needle magazine comprises a) a housing adapted to accommodate the plurality of injection needles, the housing having an exterior surface defining at least one non-circular section, b) a needle mounting space into which each of the injection needles in turn may be positioned for coupling with said associated medical delivery device, and c) an operable control member arranged at the non-circular section of the housing and being coupled or coupleable to the plurality of injection needles, the operable control member being movable relative to the non-circular section of the housing for moving the respective injection needles relative to the housing to enable the respective needles to be positioned in the needle mounting space. The operable control member comprises a belt-like structure arranged to move along the non-circular section of the housing so that respective portions of the operable control member change radius of curvature as they move along the non-circular section of the housing.

In accordance with the present invention, an operable control member shall mean an input member which may be touched directly by a finger of the user of the medical device for altering a parameter related to the operation of the medical device.

By the arrangements according to each of the first, second and third aspects, a solution is provided wherein the operable control member, regardless of the specific form-factor of the device housing, may be configured to provide a large exposed area enabling easy gripping or touching by the hands or by one or more fingers of the user. This provides for easier operation in manipulating the operable control member relative to the housing. At the same time, relative to conventional devices having a cylindrical control member, the internal space designated for use by the operable control member may be reduced offering more design freedom and ultimately enabling a more compact solution to be provided.

A guiding structure may be associated with the housing of the device, so that the guiding structure supports and guides the operable control member along its longitudinal extension along the exterior housing surfaces. The guiding structure may both comprise guiding elements to guide radially inward facing surfaces of the operable control member as well as guiding elements arranged to support the operable control elements along the edges thereof. The guiding elements may fully or partly be provided by one or more sections of the housing. The guiding structure thereby defines the shape the operable control member assumes at least along the portions of the operable control member that is exposed, i.e. that is accessible by the fingers of the user. In some forms the operable control member defines geometric surfaces adapted to engage the guiding structure to prevent the operable control member from moving in directions transverse to the direction of movement, i:e. along the longitudinal extension of the operable control member.

The guiding structure may along the length of the operable control member include stretches assuming linear sections as well as curved sections wherein parts of these sections may define paths having constant curvature.

The belt-like structure according to the first, second and third aspects may be configured to be at least partly accessible and manipulatable from the exterior of the housing of the respective devices enabling the operable control member to be touched by a user's finger along a section of the operable control member, this section being longer than 5 mm, alternatively longer than 10 mm, along the direction of movement. The operable control member is adapted to be operated manually by a user using his hand or one or more of his fingers, to cause it to move relative to the housing such as by sliding the operable control member relative to the non-circular surface section of the housing.

The operable control member may be designed to follow a path having a shape which substantially corresponds to the cross sectional shape of the surface of the housing section adjacent the operable control member, either on both adjacent sections on each side of the operable control member or, if the cross sectional shape of the two housing parts differ from each other, on a single side only.

The housing of the medical device may be so shaped and sized as to allow it to be held in a hand and to be easily carried in a pocket.

The operable control member and the adjacent housing sections on either one or on both sides of the operable control member may include a substantially flat surface (e.g. a top surface) having larger dimensions in the direction of the extension of the operable control member (i.e. along its direction of movement) as compared to the neighbouring sections of the housing (e.g. the side sections). Such relatively large top surface may be used for presenting information to the user of the device by means of a display device, such as an electronically controlled display device or by means of a mechanical display device.

Further additional aspects of the invention according to each of the first, second and third aspect of the invention will be discussed in the following.

A single or two or more operable control members may be arranged on the device as the case may be, whereby the individual operable control members may be used for altering and controlling separate respective parameters relating to the operation of the medical device.

In some embodiments, the operable control member forms an endless belt. Such endless belt may fully encircle the circumference of the device so that the operable control member may be accessible all way around the device housing. Other embodiments may include stretches which are partly covered by housing sections of the device. In still other embodiments, the operable control member is not endless but may be movable back and forth, such as between two reels.

The operable control member may be formed as a resilient band. Alternatively, it may be formed as a series of interconnected links analogous to caterpillar tracks.

Regardless the type of band or chain of links, the operable control member may be configured to form an external gripping surface having a serrated surface profile. In addition, the operable control member may be provided with visual or tactile indicia such as numbers, numbers provided as a scale, icons, directional indications such as arrows, Braille or the like. Also the operable control member may include portions of a specific colour or be coloured fully. Colouring may be used to designate the type of device for a specific parameter, such as type of medicament etc. for a medical delivery device, to differentiate the device relative to other similar devices designated other medicaments and having an operable control member in a different colour.

The operable control member in form of a flexible belt typically offers a larger grip area compared to conventional control members such as wheel type dose adjusters. The width of the belt may in some cases be increased to compose a major part of the device, resembling a way of operation analogous to a gas-handle of a motorcycle. In alternative embodiments, the operable control member forms a series of parallel raised bands which are raised relative to the adjoining housing sections. In such an embodiment, the housing section may be formed so that strips of the housing section are merged between the raised parallel bands.

The belt can be designed in many ways. The belt may for example be moulded in a polymer with a shore offering flexibility. Alternatively it can be 2K moulded by different materials simultaneously offering rigidity and flexibility.

The flexible material could be rubber or a rubber-like material. Such design will provide a relatively high friction which may offer improved grip during operation and anti-skid, e.g. the device do not skid in the hand during operation or the device do not skid when laying on a sloping surface.

The operable control member may be formed by a first material having a first set of material properties at least partially embedded in second material having a second set of material properties. At least one material property of said first and second sets of material properties differ from the corresponding material property of the other.

The first material may be a low friction material having lower friction than the second material. As parts of the operable control member made of the first material may be adapted to contact and slide against a guiding structure associated with the housing the choice of a low friction material for such parts can be used to provide increased performance as regards operability of the operable control member. The low friction material may also define structures for maintaining the operable control member in close proximity with the guiding structures. The comparatively high friction material may increase grip when touched by the hands or fingers of the user.

In addition or alternatively, sections of the operable control member made of the second material may define a radially inward facing surface and a radially outward facing surface, wherein the first material are more rigid than the second material and wherein material portions made of the first material protrudes from the radially inward facing surface and/or the radially outward facing surface. Comparatively rigid protruding elements which protrudes radially inwards may be used for enabling accurate coupling of the operable control member to components in the interior of the device, such as by mechanical engagement or to provide accurate electronic sensing of movements of the operable control member relative to the housing. Rigid protruding elements which protrude radially outwards may for example be used for visual and/or tactile indicia.

In accordance with the above mentioned second aspect, the medical delivery device of this aspect may include an operable control member which is coupled to the drive mechanism of the device so that moving the operable control member relative to the housing causes a dosage to be set for subsequent delivery of the set dose.

In certain embodiments, the operable control member itself defines a dose dial scale by having a series of dose scale markings disposed on or within surface portions of the operable control member. The operable control member may further include a thread that engages a corresponding thread associated with a dose window arranged with respect to the housing. In some embodiments the dose window is fixedly arranged relative to the housing whereby the thread of the operable control member engages a thread that is associated with the housing. In other embodiments, the window is linearly movable relative to the housing where the thread of the operable control member cooperates with a thread associated with the movable window.

In certain embodiments, the dose scale markings are arranged along a helical path on the operable control member. In such embodiments, the pitch of the helical scale markings may correspond to the pitch of the threaded connection between the operable control member and the thread associated with the window. In some embodiments, the scale markings are merged between consecutive overlapping portions of the grip areas.

In particular embodiments, the operable control member define grip areas extending in parallel with said helical path, wherein the grip areas are adapted to be exposed to be accessible from the exterior of the device to enable a user to directly manipulate the exposed portions of the operable control member.

In further embodiments the operable control member defines a minimum dose stop surface and/or a maximum dose stop surface that each are configured to cooperate with a respective counter stop surface associated with the housing to prevent the dose setting adjuster from being operated outside a predefined dose setting range.

The operable control member may also couple to a separate mechanical dose dial scale for operating the mechanical dose dial scale relative to the housing as the operable control member slides relative to the housing. Various different types of mechanical dose dial scales described in the art can be used for this purpose, e.g. one or more elements having dose indicia provided thereon. The elements may be planar, cylindrical or flexible film type scales. Also the mechanical dose dial scale may comprise one or more belt like structures having dose indicia provided thereon. Still other dose dial scales may be used such as a clock dial type.

Alternatively, or in addition, the medical delivery device may comprise an electronically controlled dose display wherein the operable control member is adapted to operate the electronically controlled dose display for setting a dose.

The operable control member, whether or not provided as a dose setting adjuster, may in certain embodiments be configured as part of a position encoder configuration which provides input to an electronic control circuit.

Various other parameters than dose setting may be designated to be controlled by the operable control member. For medical delivery devices, a non-exhaustive list of suitable parameters could include one or more of the following: a fixed dose setting operator for selecting amongst a plurality of fixed dose sizes, various parameters relating to electronic information such as time setting, navigating in a logbook of stored previously injected doses, selecting items from a list of entities etc.

Moreover, if the medical delivery device includes a needle magazine comprising a plurality of injection needles which may be used sequentially, the operable control member may be used for selecting new needles from the magazine, such as by moving the needles from respective storage positions to a needle mounting position, whereby the selected injection needle may directly mount to a needle mounting structure of the medical delivery device.

Such needle magazine may be an integrated part of the medical delivery device or may be a separate entity adapted to be coupled to the medical delivery device. In some embodiments, the needle magazine may be provided as a cap member that is adapted to releasably attach to the main housing section of the medical delivery device. The cap may then form a first part of the housing of the medical delivery device whereas the housing part that accommodates the drive mechanism may form a second part of the housing. The operable control member may be provided on one of the first and second housing parts and be adapted to operate a parameter associated with a particular function within that housing part which carries the operable control member. However, the operable control member may be adapted to operate a parameter associated with a particular function within the other housing part which does not carry the operable control member.

Still, as mentioned above, the medical delivery device may incorporate a plurality of separate operable control members each being provided in accordance with the present invention as described herein.

In a fourth aspect of the invention a medical delivery device is provided which comprises a) a housing adapted to accommodate a drug in a drug reservoir, the housing having an exterior surface defining at least one non-circular section, b) a drive mechanism for expelling doses of the drug from the drug reservoir, c) a dose setting adjuster for setting the dose to be expelled, and d) a dose dial scale comprising a series of dose amount indications arranged in a helical configuration, the dose dial scale being arranged at the non-circular section of the housing and being movable relative to the non-circular section in accordance with operation of the dose setting adjuster. The dose dial scale comprises a belt-like structure arranged to move along the non-circular section of the housing so that respective portions of the dose dial scale change radius of curvature as the respective portions move along the non-circular section of the housing. The dose dial scale further includes a thread that engages a corresponding thread associated with a dose window arranged with respect to the housing.

In a fifth aspect of the invention a medical delivery device is provided which comprises a) a housing adapted to accommodate a drug in a drug reservoir, the housing having an exterior surface defining at least one non-circular section, b) a drive mechanism for expelling doses of the drug from the drug reservoir, c) a dose setting adjuster for setting the dose to be expelled, and d) a dose dial scale comprising a series of dose amount indications, the dose dial scale being arranged at the non-circular section of the housing and being movable relative to the non-circular section in accordance with operation of the dose setting adjuster. The dose dial scale comprises a belt-like structure arranged to move along the non-circular section of the housing so that respective portions of the dose dial scale change radius of curvature as the respective portions move along the non-circular section of the housing. The dose dial scale further defines a minimum dose stop surface and/or a maximum dose stop surface that each cooperates with a respective counter stop surface associated with the housing to prevent the dose setting adjuster from being operated outside a predefined dose setting range.

In some embodiments a medical delivery device according to the fifth aspect includes a thread connection as defined in accordance with the fourth aspect.

Further embodiments of a medical delivery device having the belt-like dose dial scale according to the fourth and/or fifth aspect may include any of the further additional features discussed above in connection with the first, second and third aspects.

As used herein, the term "drug" is meant to encompass any drug-containing flowable medicine capable of being passed through a delivery means such as a hollow needle in a controlled manner, such as a liquid, solution, gel or fine suspension. Further, "drug" is meant to encompass mediums for nasal or pulmonary administration. Representative drugs include pharmaceuticals such as peptides, proteins, and hormones, biologically derived or active agents, hormonal and gene based agents, nutritional formulas and other substances in both solid (dispensed) or liquid form. In the description of the exemplary embodiments reference will be made to the use of insulin. Correspondingly, the terms "subcutaneous" and "transcutaneous" injection or infusion is meant to encompass any method of transcutaneous delivery to a subject.

### BRIEF DESCRIPTION OF THE DRAWINGS

In the following the invention will be further described with references to the drawings, wherein:
fig. 1a shows a side view representation of a first embodiment of a medical delivery device having an operable control member in accordance with the invention,
fig. 1b shows a side view similar to fig. 1a but where the housing has been partly cut providing a view of a mechanism coupled to the operable control member,
fig. 1c shows a perspective end view of the embodiment shown in figs. 1a and 1b but where an end part of the housing has been omitted,
fig. 2a and 2b show embodiments of an operable control member configuration adapted for mechanical coupling to a device mechanism,
figs. 3a and 3b show an embodiment of an operable control member configuration adapted for position encoding,
fig. 4 shows an embodiment of an operable control member providing tactile indicia,
fig. 5a is a schematic representation of a further embodiment of an operable control member configuration having a skewed grip area,
fig. 5b shows a perspective view of the operable control member shown in fig. 5a,
fig. 5c is a schematic representation of a cross sectional planar view of the operable control member configuration shown in fig. 5a,
fig. 6a shows a side view of an embodiment of a needle magazine having an operable control member and an associated medical delivery device,
fig. 6b shows a side view similar to fig. 6a but where the housing of the needle magazine has been partly cut to provide details of the needle magazine associated with the operable control member, and
fig. 6c shows a perspective view corresponding to the view shown in fig. 6b.

In the figures like structures are mainly identified by like reference numerals.

### DESCRIPTION OF EXEMPLARY EMBODIMENTS

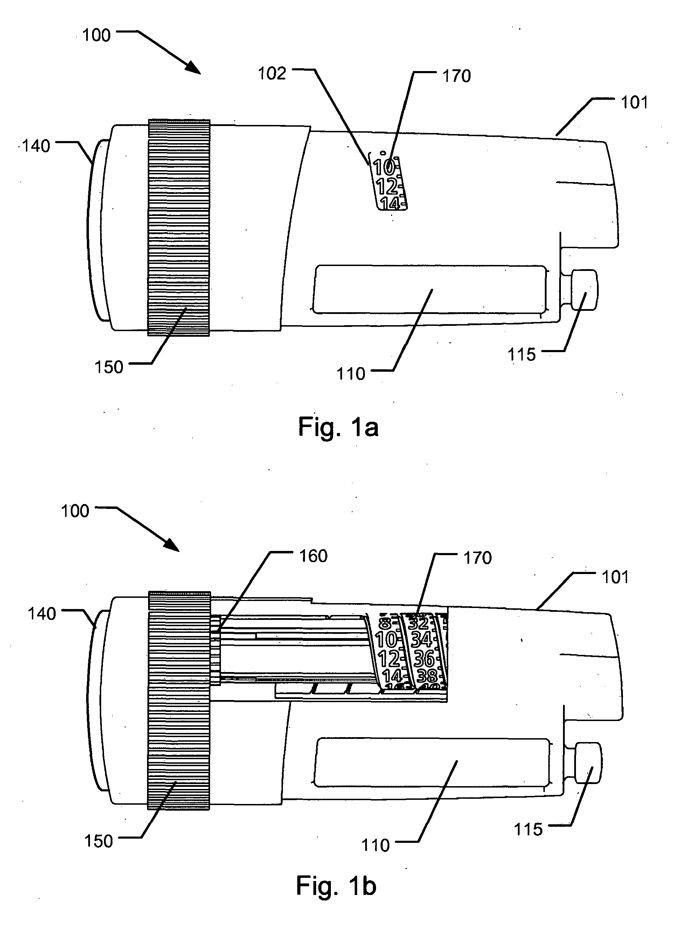

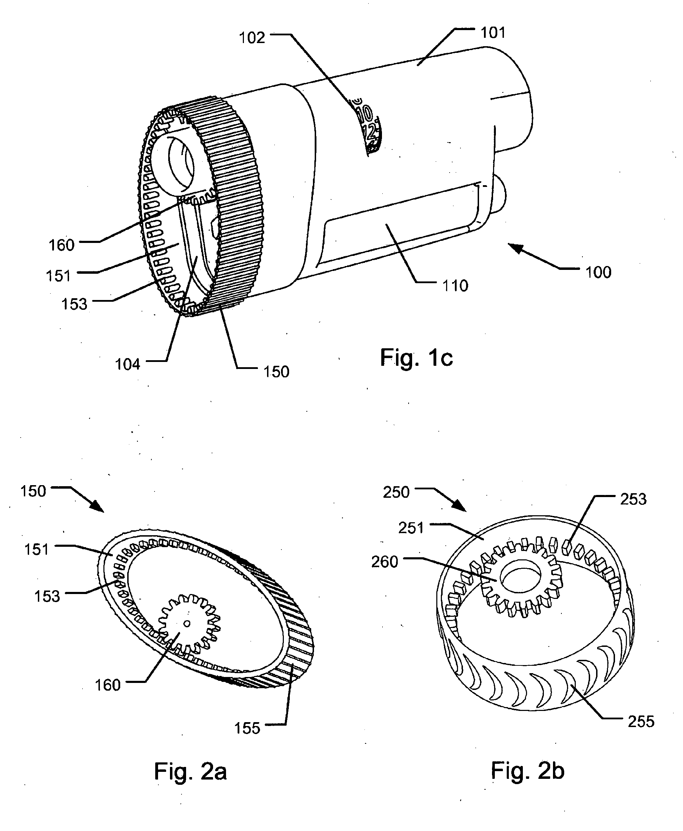

Figs. 1a-1c show different schematic representations of a first embodiment of a medical delivery device having an operable control member in accordance with an aspect of the present invention. Fig. 1 a shows a side view of a medical delivery device in the form of an injection device 100 for use by a patient for medical self treatment, the depicted device being the type of device which generally in the art is referred to as a "doser" device. The device 100 is shown as a cartridge-based device incorporating drug reservoir in the form of a piston equipped cartridge 110 having a pierceable septum which covers an outlet portion 115 of the cartridge 110. The pierceable septum is intended for cooperation with a replaceable subcutaneous needle (not shown) allowing a user to mount a fresh and sterile needle before each subcutaneous injection. At the initial stage of use, the piston of the cartridge 110 will typically be arranged at a proximal end of cartridge 110. The piston is slideably arranged in cartridge 110. In order to expel the drug contained in cartridge 110 out through the attached needle it may be forced towards the distal end, i.e. towards the outlet portion 115. The injection device may further include a cap (not shown) which detachably mounts relative to the distal end of housing 101 for protection of the contents of the cartridge 110 and eventually for protecting an injection needle which may be mounted on the device.

The injection device 100 includes a housing 101 having proximal and distal ends. The housing 101 accommodates cartridge 110 and accommodates a drive mechanism (not shown) configured to drive the piston of the cartridge 110 during an expelling operation. The drive mechanism furthermore provides for setting a dosage amount which will be expelled upon subsequent activation of an injection button 140, the injection button 140 being arranged at the proximal end of housing 101.

An operable control member in the form of a flexible band 150 encircles a section of the housing 101 at the proximal end of housing 101. In the shown embodiment the flexible band 150 forms an endless loop. The flexible band 150 is adapted to slide relative to the housing 101 along the extension of the flexible band 150. In the shown embodiment, during a dose setting process, the flexible band 150 may be manually gripped or touched by a single finger of a hand and turned in one direction relative to housing 101 away from an initial position (the zero position) in order to dial up the dose amount. In order to dial down an initially set dose the flexible band 150 may be turned in the opposite direction back towards the zero position.

The operation of the drive mechanism will not be discussed further herein but may incorporate any suitable mechanism being able to drive the piston of cartridge 110 towards the outlet portion 115 of cartridge 110. One non-exhaustive example of a drive mechanism suitable to be used in medical injection device 100 and in combination with the operable control member configuration in accordance with the present invention is described in WHO 98/57688, this reference being incorporated herein in its entirety.

Housing 101 further includes a window or opening 102 which provides visual inspection to a mechanically based dose dial scale 170 arranged internally in housing 101. Dose dial scale 170 includes various printed indicia, such as numerals, printed thereon, each indicia corresponding to the respective dose sizes that the dose setting part of drive mechanism is designed to assume. As will be described below, the dose dial scale 170 is operated by means of flexible band 150 as it is turned relative to the housing 101 during the dose setting process.

Fig. 1b shows a further side view of injection device 100 where a part of the housing 101, for illustrative purposes, has been cut away to allow partial inspection of dose dial scale 170. Fig. 1b further reveals a gear wheel 160 which rotatably couples to dose dial scale 170 in a manner so that these two components rotate together. In the depicted form, the dose dial scale 170 is provided as a helical type dose dial scale which is so mounted that it moves axially as it is rotated relative to housing 101. Hence, each single dose indicia on dose dial scale 170 may be aligned with window 102 in accordance with the dose set by means of the flexible band 150.

Fig. 1c shows a proximal perspective end view of injection device 101. In fig. 1c, the injection button 140 and the most proximal part of housing 101 have been omitted in the drawing to allow inspection of details relating to the flexible band 150 and its mechanical coupling to the dose dial scale 170. Flexible band 150 defines a radially inwards facing surface which defines a number of teeth 153 adapted to cooperate with mating teeth of gear wheel 160. As the flexible band 150 slides relative to the housing 101, the teeth of the flexible band 150 induces a rotational movement of gear wheel 160 accompanied by a corresponding rotational movement of dose dial scale 170. Hence, a desired dose size is easily selected by way of turning flexible band relative to housing 101.

In alternative embodiments, the dose dial scale may be provided as an odometer type scale having separately movable rings which in combination provides the various dose amount indications. Also, the flexible disc type dose dial scale disclosed in WO 01/87386 may be incorporated in a medical delivery according to this invention. Furthermore, electronically based dose dial indicators may alternatively be used.

The drive mechanism may be coupled to dose dial scale 170 or gear wheel 160 so that the rotational movements of dose dial scale 170 cause a dose setting adjustment of the drive mechanism of device 100. Alternatively, a separate gear wheel (not shown) additionally couples to teeth 153 of the flexible band 150 so that the dose setting adjustment of the drive mechanism is transmitted directly from flexible band 150.

Fig. 1c additionally reveals the form-factor of the injection device 100. In the depicted embodiment, the proximal section of housing 101 is designed having a cross sectional shape which is approximately oval. The housing sections adjacent the flexible band 150 include guiding structures 104 adapted to support and guide the flexible band 150 relative to housing 101, e.g. by guiding the interior facing surface 151 of flexible band 150 and/or the edge sections thereof.

Fig. 2a shows a more detailed view of flexible band 150. The internal teeth 153 of flexible band 150 are molded in one piece and integral with the remaining parts of the flexible band 150. The external surface of flexible band 150 comprises a plurality of protruding ribs 154 which offer increased grip during operation of the flexible band 150 during the dose setting process.

Fig. 2b shows a second embodiment of an operable control member according to an aspect of the invention suitable for use in a medical device. Again, the operable control member is formed as a flexible band 250 which here is depicted in its relaxed state assuming a circular shape. Also shown is a transmission gear wheel 260 cooperating with internal teeth 253. External ribs are formed in a shape offering directional guidance to the user for signaling a particular direction of movement. Also in this embodiment, the flexible band 250, when inserted into the final end-product, may be adapted to assume a shape corresponding to the desired form factor of the device which typically will be non-circular. Again, the shape of the flexible band generally corresponds to a guiding structure associated with the device housing of the particular medical device in question wherein the guiding structure engages an internal facing surface 251 of flexible band 250 and possibly also the edge sections of flexible band 250.

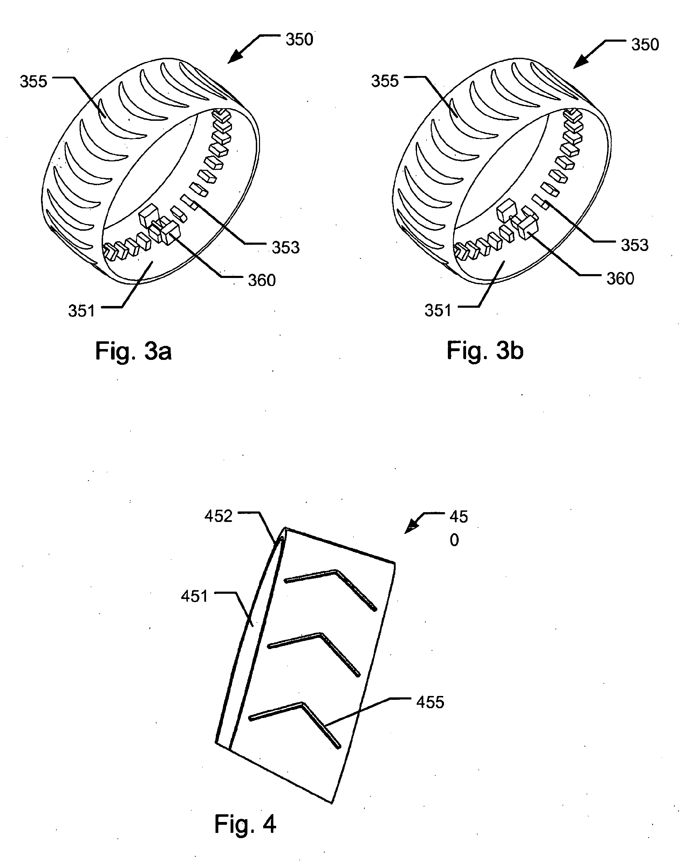

Figs. 3a and 3b show a further embodiment of a flexible band 350 which is shown in an operable control member configuration wherein the internal teeth 353 is adapted to cooperate with a position sensing arrangement adapted to detect positional movement of the flexible band 350 relative to a housing section of the particular medical device that flexible band 350 is incorporated into. In this example, an optocoupling device is arranged to sense the internal teeth 353 so that one or more emitted light beams senses the number of teeth 353 that passes between an emitter and a receiver of the optocoupling device. Such arrangement may for example be used in a medical delivery device, such as a motorized doser, where the operable control member is part of a position encoder configuration which provides input to an electronic control circuit. The flexible band 350 may for example be adapted to monitor or control a dose setting process. Other suitable applications may include the flexible band 350 being used for adjusting other parameters of the device, such as during the setting of an internal clock, browsing through a logbook of stored dose information etc. Other types of position encoders may alternatively be used utilizing principles other than optocoupling, such as principles using reflective optical signals, magnetic fields, inductive fields, capacitive fields or electrical galvanic switch signals which either cooperate with elements formed integral with or on the flexible band 350. Alternative position encoders may also be used which cooperate or associates with an element in mechanical engagement with teeth 353 or other structural members associated with flexible band 350.

Fig. 4 shows a further embodiment of a flexible band 450 which are formed by a 2k injection molding process. Here the base material of flexible band 450 may be made of rubber having a number of injected molded pieces 455 partly embedded therein. The in-molded pieces may provide a greater stiffness than corresponding structures made of the material of the base material. Here ribs 455 provide both additional grip friction during operation and provide additional tactile and visual indication of the ribs. The visual indication may be further improved if the colors of the two materials differ from each other. Additional in-molded structures 452, one of which is shown in fig. 4, may be provided to obtain further mechanical advantages. For example, in-molded structures on the interior face 451 and/or along the edge sections of flexible band 450 may be provided, the in-molded structures being adapted to slide against the guidance structures of the housing of the associated medical device. In such configurations, the material of the additional in-molded structures 452 may provide a reduced friction compared to the friction of the base material of the flexible band 450 and hence provides for a flexible band structure which offers a smooth sliding movement of flexible band 450 relative to the device housing of the associated medical device.

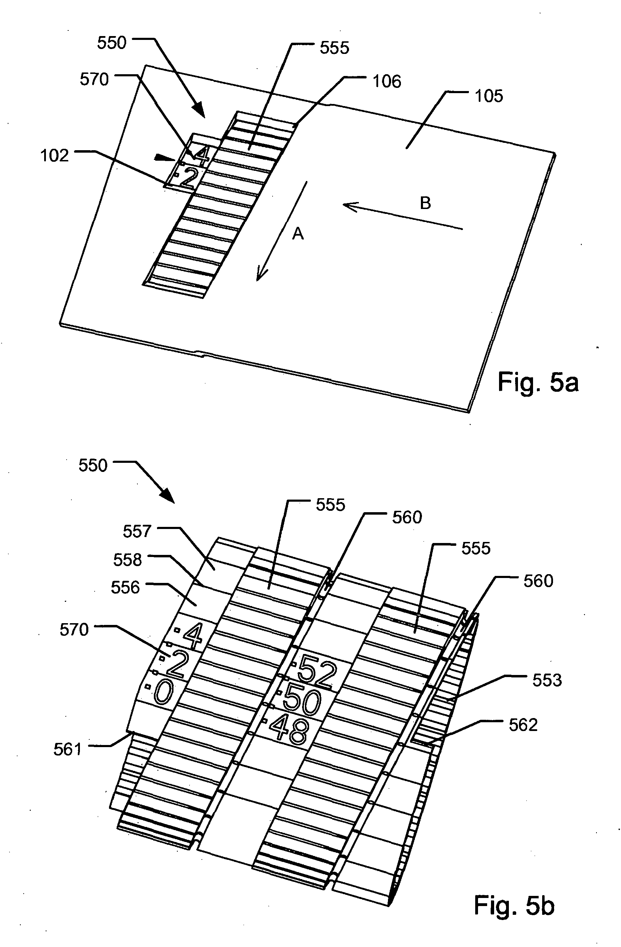

Fig. 5a through fig. 5c show schematic representations of a further embodiment of an operable control member configuration in a medical delivery device which incorporates an operable control member 550 configured as a dose setting operator having a mechanical dose dial scale 570 associated therewith. Fig. 5a schematically shows part of a housing section 105 which includes a cut-away portion 106 adapted to expose a portion of the operable control member 550 allowing the user of the associated medical device to operate the operable control member 550 by manually forcing the operable control member 550 along the arrow indicated "A" relative to the housing section 105. The housing section 105 further comprises a window or opening 102 which exposes one or more of the symbols forming the dose dial scale 570 for visual inspection.

Fig. 5b shows a perspective view of operable control member 550. Operable control member 550 is in the depicted embodiment formed as an endless belt comprising a series of articulated links 556, 557 that are interconnected by joints 558 so that each pair of neighboring links 556, 557 may swivel relative to each other around an axis defined by the joints 558, said axis being parallel to the arrow "B" shown in fig. 5a. When assembled in the medical device the endless belt assumes a flat configuration. Operable control member 550 defines a grip area 555 that extends circumferentially in a substantial helical configuration approximately two times the circumference of the endless belt. As apparent from figs. 5a and 5b, the grip area 555 is skewed relative to a direction orthogonal to the axis defined by the joints 558.

The internal parts of the endless belt exhibits a number of teeth 153 adapted to cooperate with mating teeth of a gear wheel (not shown) of a drive mechanism (also not shown) to mechanically transmit the movements of the operable control member 550 relative to housing section 105 to the drive mechanism.

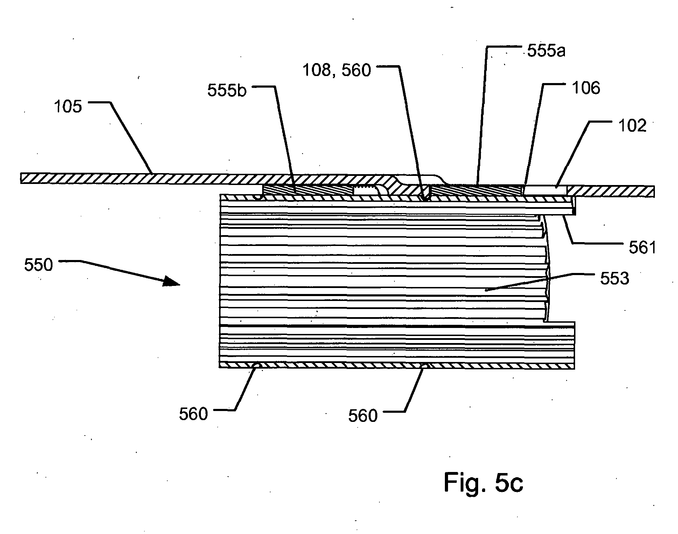

The operable control member 550 further defines a thread 560 running along the skewed grip area 555, e.g. running in parallel. Thread 560 engages cooperating thread sections (108, see fig. 5c) of a guiding structure formed in or associated with housing section 105. In addition, or alternatively, the side edges of the grip area 555 cooperate with a guiding structure associated with the housing section 105.

The dose dial scale 570 is disposed in a band-like fashion next to the grip area 555 and hence extends parallel to the grip area 555 merged between consecutive overlapping sections of the grip area. In accordance with the particular relative position between operable control member 550 and housing section 105, selected ones of the symbols of the dose dial scale 570 are viewable through window or opening 102.

From the embodiment shown in figs. 5a, 5b and 5c it is clear that the housing section 105 only exposes a part of the grip area 555a so that only a strip section of grip area 555 is configured to be directly touched by the user. Other parts 555b of grip area are hidden inside the housing of the device.

Due to the skewed configuration of the thread 560 and the grip area 555, when the operable control member 550 is operated in the direction along arrow "A" the operable control member 550 is moved along arrow "B" and the corresponding symbol of dose dial scale 570 shows up in the window or opening 102. Compared to conventional cylindrical dose dial scales such as the one shown in fig. 1a and 1b, a dose dial scale such as the dose dial scales 570 described herein enables use of a larger font size for the symbols of the dose dial scale and hence offers increased visibility.

Fig. 5b and 5c further show a minimum dose stop surface 561 which extends substantially parallel with the axis of the joints 558. When the operable control member 550 is adjusted to assume its minimum dose size setting, i.e. typically where the zero dose indication is aligned in dose window 102, a corresponding minimum dose counter stop surface (not shown) associated with the housing section 105 engages the minimum dose stop surface 561 to prevent the operable control member 550 from moving below that particular dose setting. Fig. 5b further shows a similar maximum dose stop surface 562 which is adapted to engage a corresponding maximum dose counter stop surface (not shown) associated with the housing section 105. This prevents the operable control member 550 from moving beyond a particular maximum dose setting designated for the specific application. It is noted that in applications where the operable control member 550 returns from a set desired dose to the minimum dose setting as the set dose is expelled from the device, the minimum dose stop surface 561 and its corresponding minimum dose counter stop surface may act as an end of dose limiter assuring a well-defined end of dose stop at the end of the dose expelling procedure.

In other embodiments where a dose window is arranged movably relative to the housing sections of the delivery device, the threaded sections of the operable control member may be engaging corresponding thread sections of the movable dose window so that the dose window follows the helically arranged dose dial scale as the operable control member is operated.

It is to be noted that in accordance with the fourth and fifth aspects of the invention as referred to above, further embodiments of a dose dial configuration provided as a flexible belt similar to the operable control member 550 described above but where grip areas are omitted, may be envisioned as well. Such flexible belt dose dial configuration will typically be incorporated within the device so that no parts of the dose dial scale will be accessible by the fingers of a user.

In accordance with the fourth aspect, such flexible belt dose dial configuration will have a thread associated with the flexible belt that cooperates with a corresponding thread associated with a dose window. The threaded engagement ensures that a helically arranged dose dial scale will be moved relative to the dose window both along a main axis of movement (cf. arrow "A" in fig. 5a) as well as in directions transverse to the main axis of movement (cf. arrow "B" of fig. 5a) as the dose dial is operated. Still other alternatives-employ a cam and follower engagement replacing the thread connection and enabling step wise transverse movements between the dose dial scale and the dose window, e.g. generally in accordance with the principles set forth in WO 2008/148864.

In accordance with the fourth and fifth aspects, the medical delivery device may include a dose setting adjuster, such as a turnable knob which is coupled to the flexible belt dose dial for operation of the flexible belt dose dial and a dose setting arrangement of the medical delivery device.

Also, in accordance with the fifth aspect, such dose dial configuration, optionally in combination with the fourth aspect, will define a minimum dose stop surface and/or a maximum dose stop surface that cooperates with respective counter stop surfaces associated with the housing in a manner generally similar to the stop surfaces defined by elements 561 and 562 described above.

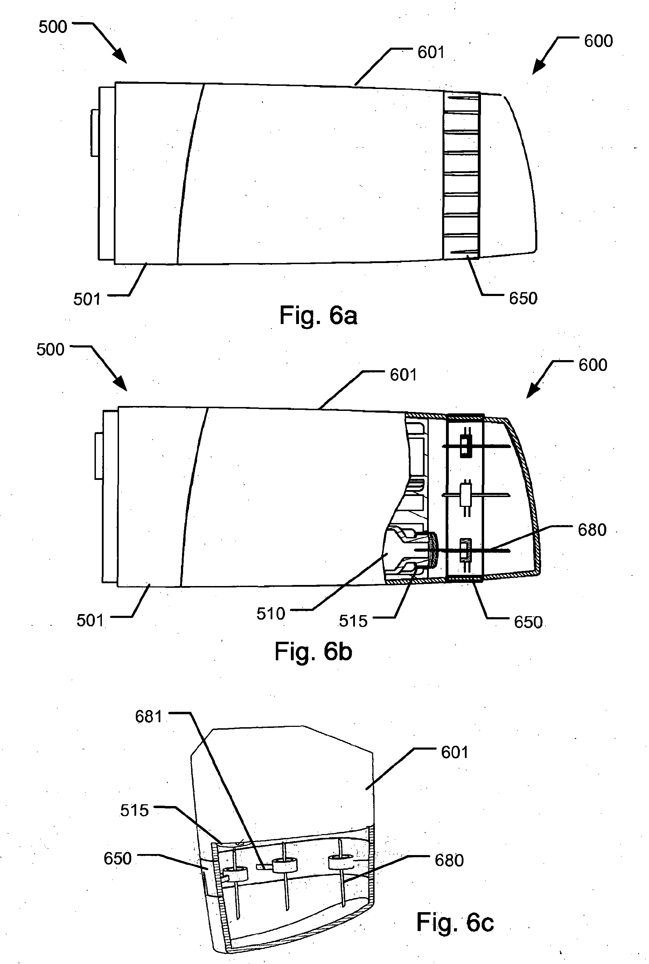

Figs. 6a through fig. 6c show schematic representations of a further embodiment according to the invention. In fig. 6a, a medical delivery device 500 in the form of a dosing device is adapted to cooperate with a needle magazine 600. The needle magazine 600 is integrated in a cap defining a housing 601 which releasably attaches to the housing 501 of the medical delivery device so that the cap protects the distal end of the medical delivery device 500. As shown in figs. 6b and 6c, the needle magazine 600 accommodates a plurality of injection needles 680 which in turn may be used during separate dose administrations. The needle magazine 600 includes an operable control member 650 which generally works in a similar way as described in the above discussed embodiments. The shown embodiment of the needle magazine 600 and the delivery device 500 both assume a generally non-circular shape, e.g. a non-circular cross section and the operable control member 650 generally assumes a shape corresponding to the neighboring sections of housing 601. A radially inwards facing surface of the operable control member 650 includes a series of holding structures 681 each adapted to hold an injection needle 680. By operating the operable control member 650 by means of a sliding movement relative to housing 601 each of the injection needles 680 is moved along the path of the operable control member 650. In this way, the injection needles are brought from a storage position and into a needle mounting position which may be defined as a needle mounting space, ready for connection to the outlet end 515 of the cartridge 510. It is to be noted that the housing 601 of the needle magazine may require to be moved axially towards or away from the medical delivery device 500 for attachment or detachment of the particular injection needle 680 relative to the outlet of the cartridge 510 to be facilitated. Once a new injection needle 680 has been selected by means of operable control member 650, that particular needle is mounted onto a mounting structure of the medical delivery device 500 and the needle magazine 600 may then be detached from the medical delivery device 500 thus making the medical delivery device 500 ready for performing an injection.

Other non-exhaustive examples of needle magazines suitable to be used in combination with the operable control member of the present invention is described in WO 2009/016161, US 5,829,589 and US 2002020646. In such needle magazines, the operable control member is adapted to move relative to a housing section of the needle magazine and may be adapted to move the plurality of needles of the magazine, either simultaneously or one by one, so that respective ones of the needles are brought from a storage position and into a needle mounting position in a needle mounting space of the needle magazine. In accordance with the discussion above, the said housing section of the needle magazine may be made non-circular.

In line with the invention as set forth above, the invention is generally applicable to medical delivery devices, regardless of the kind of administration route for delivering a beneficial agent to the user. Also, the invention may be implemented in both injection devices of the non-motorized kind as well as motorized dosers. As regards non-motorized injection devices, such devices both encompass manual injectors where the user directly delivers the necessary mechanical energy during the delivery process as well as spring assisted injectors where a pre-stressed or user strained spring in part or fully delivers the necessary mechanical energy during the delivery process. Further, the invention may be adopted to be used in connection with other medical equipment for controlling a parameter associated with the operation of that particular medical device.

In the above description of the exemplary embodiments, the different structures providing the desired relations between the different components just as the means providing the described functionality for the different components have been described to a degree to which the concept of the present invention will be apparent to the skilled reader. The detailed construction and specification for the different structures are considered the object of a normal design procedure performed by the skilled person along the lines set out in the present specification.

## Claims

1. A medical delivery device (100) comprising:
- a housing (101, 105) adapted to accommodate a drug in a drug reservoir (110), the housing having an exterior surface defining at least one non-circular section,
- a drive mechanism for expelling doses of the drug from the drug reservoir (110),
- an operable control member (150, 250, 350, 450, 550, 650) directly manipulatable by a user and arranged at the non-circular section of the housing (101, 105), the operable control member (150, 250, 350, 450, 550, 650) being movable relative to the non-circular section of the housing (101, 105) for altering a parameter relating to the operation of the medical delivery device (100), **characterized in that** the operable control member (150, 250, 350, 450, 550, 650) comprises a belt-like structure arranged to move along the non-circular section of the housing (101, 105) and wherein respective portions of the operable control member (150, 250, 350, 450, 550, 650) change radius of curvature as they move along the non-circular section of the housing (101, 105).

2. A medical delivery device as defined in claim 1, wherein the operable control member (150, 250, 350, 450, 550, 650) forms an endless belt.

3. A medical delivery device as defined in claims 1 or 2, wherein the operable control member (150, 250, 350, 450, 550, 650) is formed as a resilient band.

4. A medical delivery device as defined in claims 1 or 2, wherein the operable control member (150, 250, 350, 450, 550, 650) is provided as a series of interconnected links (556, 557).

5. A medical delivery device as defined in any the previous claims, wherein the operable control member (150, 250, 350, 450, 550, 650) forms an external gripping surface (555) having a serrated surface profile.

6. A medical delivery device as defined in any of the previous claims, wherein the operable control member (150, 250, 350, 450, 550, 650) circumscribes the non-circular section of the housing (101, 105) and is manually gripable all the way around the non-circular section of the housing (101, 105).

7. A medical delivery device as defined in any of the previous claims, wherein the operable control member (150, 250, 350, 450, 550, 650) is coupled to the drive mechanism so that moving the operable control member (150, 250, 350, 450, 550, 650) relative to the housing causes a dosage to be set for subsequent delivery of the set dose.

8. A medical delivery.device as defined in claim 7, wherein the operable control member (150, 250, 350, 450, 550, 650) couples to a mechanical dose dial scale (170, 570) for operating the mechanical dose dial scale (170, 570) relative to the housing (101, 105).

9. A medical delivery device as defined in claim 8 , wherein the mechanical dose dial scale (170, 570) comprises one or more belt like structures (556, 557) having dose indicia provided thereon.

10. A medical delivery device as defined in any of the previous claims, wherein the operable control member (150, 250, 350, 450, 550, 650) is provided with indicia.

11. A medical delivery device as defined in claim 10, wherein the indicia forms a dose dial scale provided on surface portions of the belt-like structure (556, 557).

12. A medical delivery device as defined in any of the previous claims, wherein the medical delivery device (100) further comprises an electronically controlled dose display and wherein the operable control member (150, 250, 350, 450, 550, 650) is adapted to operate the electronically controlled dose display.

13. A medical delivery device as defined in any of the previous claims, wherein the operable control member (150, 250, 350, 450, 550, 650) is formed by a first material having a first set of material properties at least partially embedded in second material having a second set of material properties and wherein at least one material property of said first and second sets of material properties differ from the corresponding material property of the other.

14. A medical delivery device as defined in claim 13, wherein the first material is a low friction material having lower friction than the second material and wherein sections (151, 251, 560) of the operable control member made of the first material are adapted to contact and slide against a guiding structure (104, 108) associated with the housing (101, 105).

15. A medical delivery device as defined in claims 13 or 14, wherein sections of the operable control member (150, 250, 350, 450, 550, 650) made of the second material defines a radially inward facing surface and a radially outward facing surface, wherein the first material is more rigid than the second material and wherein material portions (452, 455) made of the first material protrudes from the radially inward facing surface and/or the radially outward facing surface.

## Patentansprüche

1. Medizinische Verabreichungsvorrichtung (100), umfassend:
- ein Gehäuse (101, 105) zur Aufnahme eines Arzneimittels in einem Arzneimittelreservoir (110), wobei das Gehäuse eine Außenfläche aufweist, die zumindest einen nicht-kreisförmigen Abschnitt definiert,
- einen Antriebsmechanismus zum Ausstoßen von Dosierungen des Arzneimittels aus dem Arzneimittelreservoir (110),
- ein bedienbares Steuerelement (150, 250, 350, 450, 550, 650), das von einem Anwender direkt manipuliert werden kann und an dem nicht-kreisförmigen Abschnitt des Gehäuses (101, 105) angeordnet ist, wobei das bedienbare Steuerelement (150, 250, 350, 450, 550, 650) zur Änderung eines Parameters, der sich auf die Bedienung der medizinischen Verabreichungsvorrichtung (100) bezieht, relativ zu dem nicht-kreisförmigen Abschnitt des Gehäuses (101, 105) bewegbar ist, **dadurch gekennzeichnet, dass** das bedienbare Steuerelement (150, 250, 350, 450, 550, 650) eine riemenartige Konstruktion umfasst, die ausgelegt ist, sich entlang des nicht-kreisförmigen Abschnitts des Gehäuses (101, 105) zu bewegen, und worin jeweilige Abschnitte des bedienbaren Steuerelements (150, 250, 350, 450, 550, 650) bei ihrer Bewegung entlang des nicht-kreisförmigen Abschnitts des Gehäuses (101, 105) ihren Krümmungsradius ändern.

2. Medizinische Verabreichungsvorrichtung nach Anspruch 1, worin das bedienbare Steuerelement (150, 250, 350, 450, 550, 650) einen Endlosriemen bildet.

3. Medizinische Verabreichungsvorrichtung nach Anspruch 1 oder 2, worin das bedienbare Steuerelement (150, 250, 350, 450, 550, 650) als elastisches Band geformt ist.

4. Medizinische Verabreichungsvorrichtung nach Anspruch 1 oder 2, worin das bedienbare Steuerelement (150, 250, 350, 450, 550, 650) als eine Reihe von miteinander verbundenen Verbindungsgliedern (556, 557) bereitgestellt ist.

5. Medizinische Verabreichungsvorrichtung nach einem der vorhergehenden Ansprüche, worin das bedienbare Steuerelement (150, 250, 350, 450, 550, 650) eine äußere Greiffläche (555) mit einem geriffelten Oberflächenprofil bildet.

6. Medizinische Verabreichungsvorrichtung nach einem der vorhergehenden Ansprüche, worin das bedienbare Steuerelement (150, 250, 350, 450, 550, 650) den nicht-kreisförmigen Abschnitt des Gehäuses (101, 105) umschreibt und rings um den nicht-kreisförmigen Abschnitt des Gehäuses (101, 105) manuell greifbar ist.

7. Medizinische Verabreichungsvorrichtung nach einem der vorhergehenden Ansprüche, worin das bedienbare Steuerelement (150, 250, 350, 450, 550, 650) mit dem Antriebsmechanismus verbunden ist, so dass eine Bewegung des bedienbaren Steuerelements (150, 250, 350, 450, 550, 650) relativ zu dem Gehäuse die Einstellung einer Dosis für die anschließende Abgabe der eingestellten Dosis verursacht.

8. Medizinische Verabreichungsvorrichtung nach Anspruch 7, worin das bedienbare Steuerelement (150, 250, 350, 450, 550, 650) zur Bedienung der mechanischen Dosisskala (170, 570) relativ zu dem Gehäuse (101, 105) mit einer mechanischen Dosisskala (170, 570) verbunden ist.

9. Medizinische Verabreichungsvorrichtung nach Anspruch 8, worin die mechanische Dosisskala (170, 570) eine oder mehrere riemenartige Konstruktionen (556, 557) mit darauf bereitgestellten Dosismarkierungen umfasst.

10. Medizinische Verabreichungsvorrichtung nach einem der vorhergehenden Ansprüche, worin das bedienbare Steuerelement (150, 250, 350, 450, 550, 650) mit Markierungen ausgestattet ist.

11. Medizinische Verabreichungsvorrichtung nach Anspruch 10, worin die Markierungen eine Dosisskala bilden, die auf Oberflächenabschnitten der riemenartigen Konstruktion (556, 557) bereitgestellt sind.

12. Medizinische Verabreichungsvorrichtung nach einem der vorhergehenden Ansprüche, worin die medizinische Verabreichungsvorrichtung (100) ferner eine elektronisch gesteuerte Dosisanzeige umfasst und worin das bedienbare Steuerelement (150, 250, 350, 450, 550, 650) zur Bedienung der elektronisch gesteuerten Dosisanzeige ausgelegt ist.

13. Medizinische Verabreichungsvorrichtung nach einem der vorhergehenden Ansprüche, worin das bedienbare Steuerelement (150, 250, 350, 450, 550, 650) von einem ersten Material mit einem ersten Satz von Materialeigenschaften geformt wird, das zumindest teilweise in einem zweiten Material mit einem zweiten Satz von Materialeigenschaften eingebettet ist, und worin sich zumindest eine Materialeigenschaft der ersten und zweiten Sätze von Materialeigenschaften von der entsprechenden Materialeigenschaft der anderen unterscheidet.

14. Medizinische Verabreichungsvorrichtung nach Anspruch 13, worin das erste Material ein reibungsarmes Material mit geringerer Reibung als das zweite Material ist und worin Abschnitte (151, 251, 560) des bedienbaren Steuerelements, die aus dem ersten Material bestehen, ausgelegt sind, eine mit dem Gehäuse (101, 105) in Zusammenhang stehende Führungskonstruktion (104, 108) zu berühren und zu dieser zu gleiten.

15. Medizinische Verabreichungsvorrichtung nach Anspruch 13 oder 14, worin Abschnitte des bedienbaren Steuerelements (150, 250, 350, 450, 550, 650), die aus dem zweiten Material bestehen, eine radial nach innen weisende Fläche und eine radial nach außen weisende Fläche definieren, worin das erste Material starrer als das zweite Material ist und worin Materialabschnitte (452, 455), die aus dem ersten Material bestehen, von der nach radial nach innen weisenden Fläche und/oder der radial nach außen weisenden Fläche vorstehen.

## Revendications

1. Dispositif médical de distribution (100), comprenant:
- un boîtier (101, 105) adapté pour contenir un médicament dans un réservoir de médicament (110), le boîtier comprenant une surface extérieure définissant au moins une section non circulaire,
- un mécanisme d'entraînement pour expulser des doses du médicament à partir du réservoir de médicament (110) ,
- un élément de commande actionnable (150, 250, 350, 450, 550, 650) pouvant être manipulé directement pas un utilisateur et agencé à la section non circulaire du boîtier (101, 105), l'élément de commande actionnable (150, 250, 350, 450, 550, 650) étant mobile par rapport à la section non circulaire du boîtier (101, 105) pour modifier un paramètre relatif au fonctionnement du dispositif médical de distribution (100),
**caractérisé en ce que** l'élément de commande actionnable (150, 250, 350, 450, 550, 650) comprend une structure de type courroie agencée de manière à se déplacer le long de la section non circulaire du boîtier (101, 105), et dans lequel des parties respectives de l'élément de commande actionnable (150, 250, 350, 450, 550, 650) changent de rayon de courbure lorsqu'elles se déplacent le long de la section non circulaire du boîtier (101, 105).

2. Dispositif médical de distribution selon la revendication 1, dans lequel l'élément de commande actionnable (150, 250, 350, 450, 550, 650) forme une courroie sans fin.

3. Dispositif médical de distribution selon la revendication 1 ou 2, dans lequel l'élément de commande actionnable (150, 250, 350, 450, 550, 650) est formé comme une bande élastique.

4. Dispositif médical de distribution selon la revendication 1 ou 2, dans lequel l'élément de commande actionnable (150, 250, 350, 450, 550, 650) est fourni comme une série de liens interconnectés (556, 557).

5. Dispositif médical de distribution selon l'une quelconque des revendications précédentes, dans lequel l'élément de commande actionnable (150, 250, 350, 450, 550, 650) forme une surface d'accrochage externe (555) présentant un profil de surface dentelé.

6. Dispositif médical de distribution selon l'une quelconque des revendications précédentes, dans lequel l'élément de commande actionnable (150, 250, 350, 450, 550, 650) entoure la section non circulaire du boîtier (101, 105) et peut être agrippé manuellement tout autour de la section non circulaire du boîtier (101, 105) .

7. Dispositif médical de distribution selon l'une quelconque des revendications précédentes, dans lequel l'élément de commande actionnable (150, 250, 350, 450, 550, 650) est couplé au mécanisme d'entraînement de telle sorte qu'un déplacement de l'élément de commande actionnable (150, 250, 350, 450, 550, 650) par rapport au boîtier entraîne qu'un dosage soit réalisé en vue d'une distribution subséquente de la dose déterminée.

8. Dispositif médical de distribution selon la revendication 7, dans lequel l'élément de commande actionnable (150, 250, 350, 450, 550, 650) se couple à un compteur mécanique à cadran de doses (170, 570) pour actionner le compteur mécanique à cadran de doses (170, 570) par rapport au boîtier (101, 105).

9. Dispositif médical de distribution selon la revendication 8, dans lequel le compteur mécanique à cadran de doses (170, 570) comprend une ou plusieurs structure(s) de type courroie (556, 557) comportant des indications de doses prévues sur celle(s)-ci.

10. Dispositif médical de distribution selon l'une quelconque des revendications précédentes, dans lequel l'élément de commande actionnable (150, 250, 350, 450, 550, 650) comporte des indications.

11. Dispositif médical de distribution selon la revendication 10, dans lequel les indications forment un compteur à cadran de doses prévu sur des parties de surface de la structure de type courroie (556, 557).

12. Dispositif médical de distribution selon l'une quelconque des revendications précédentes, dans lequel le dispositif médical de distribution (100) comprend en outre un écran d'affichage de doses commandé électroniquement, et lequel l'élément de commande actionnable (150, 250, 350, 450, 550, 650) est apte à activer l'écran d'affichage de dose commandé électroniquement.

13. Dispositif médical de distribution selon l'une quelconque des revendications précédentes, dans lequel l'élément de commande actionnable (150, 250, 350, 450, 550, 650) est formé à partir d'un premier matériau qui présente un premier ensemble de propriétés de matériau au moins partiellement incorporées dans un deuxième matériau qui présente un deuxième ensemble de propriétés de matériau, et dans lequel au moins une propriété de matériau desdits premier et deuxième ensembles de propriétés de matériau diffèrent de la propriété de matériau correspondante de l'autre.

14. Dispositif médical de distribution selon la revendication 13, dans lequel le premier matériau est un matériau à faible coefficient de frottement dont le frottement est inférieur à celui du deuxième matériau, et dans lequel des sections (151, 251, 560) de l'élément de commande actionnable constituées du premier matériau sont aptes à entrer en contact avec et à coulisser contre une structure de guidage (104, 108) associée audit boîtier (101, 105).

15. Dispositif médical de distribution selon la revendication 13 ou 14, dans lequel des sections de l'élément de commande actionnable (150, 250, 350, 450, 550, 650) constituées du deuxième matériau définissent une surface orientée radialement vers l'intérieur et une surface orientée radialement vers l'extérieur, dans lequel le premier matériau est plus rigide que le deuxième matériau, et dans lequel des parties de matériau (452, 455) constituées du premier matériau font saillie à partir de la surface orientée radialement vers l'intérieur et/ou de la surface orientée radialement vers l'extérieur.
